# EUROPEAN PATENT APPLICATION

(11) **EP 4 368 180 A1**
(43) Date of publication of application: **15.05.2024**
(21) Application number: 22837064.9
(22) Date of filing: 08.07.2022
(51) Int. Cl.: A61K 31/192, A61P 27/02, A61P 29/00

(54) **APPLICATION OF LOXOPROFEN SODIUM IN PREPARATION OF DRUG FOR TREATING DRY EYE DISEASE**

(30) Priority: 09.07.2021 CN 202110780762
(71) Applicant: Guangzhou Ocusun Ophthalmic Biotechnology Co., Ltd., Guangzhou, Guangdong 511400 (CN); Ocusun Ophthalmic Pharmaceutical (Guangzhou) Co., Ltd., Guangzhou, Guangdong 511400 (CN)
(72) Inventor: LIANG, Chiyong, Guangzhou, Guangdong 511400 (CN); WANG, Yandong, Guangzhou, Guangdong 511400 (CN); YU, Chuiliang, Guangzhou, Guangdong 511400 (CN); LI, Liang, Guangzhou, Guangdong 511400 (CN); XUE, Yaping, Guangzhou, Guangdong 511400 (CN); WU, Meirong, Guangzhou, Guangdong 511400 (CN)
(74) Representative: Murgitroyd & Company
(86) International application number: PCT/CN2022/104727
(87) International publication number: WO 2023/280319

(57) **Abstract**

The present invention relates to an application of loxoprofen sodium or a composition containing loxoprofen sodium in the preparation of a drug for preventing and/or treating the dry eye disease.

## Description

The present application claims the priority of Chinese Patent Application No. 202110780762.8, filed on July 09, 2021, which is incorporated herein by reference in its entirety.

### TECHNICAL FIELD

The present invention relates to the field of medicines, and in particular to a use of loxoprofen sodium or a composition containing loxoprofen sodium in the preparation of a drug for preventing and/or treating a dry eye disease.

### BACKGROUND

The dry eye disease is also known as keratoconjunctivitis sicca syndrome. The Tear Film & Ocular Surface Society (TFOS) has redefined the dry eye disease at Dry Eye Workshop II in 2015, that is: the dry eye disease (DED) is an ocular multi-factorial disease. It is characterized by a loss of homeostasis of the tear film accompanied by ocular surface symptoms which are mainly dry eyes, foreign body sensation, and burning, often accompanied by sticky secretions in the eyes. Due to poor sleep quality, excessive use of eyes, a person with a severe case can even be affected in vision and normal life.

In recent years, the incidence of the dry eye disease has been increasing year by year, and it has become one of the most common ophthalmic diseases. The incidence of the dry eye disease is 5.5-33.7% worldwide. The incidence is higher in women than in men, higher in the elderly than in young people, and higher in Asians than people in other regions. Foreign epidemiological reports show that the prevalence rate of the dry eye disease is 20% in people aged 30 to 40 years old; among people over 70 years old, the prevalence rate of the dry eye disease reaches up to 36.1%; and in these people, the prevalence rate of the dry eye disease in women is still higher than that in men.

There are many causes of the dry eye disease, including their own factors such as damaged tear films, lacrimal gland dysfunction, meibomian gland dysfunction, abnormal expression of ocular surface proteins, vitamin A deficiency, sex hormone disorders, and abnormal neuromodulation. Many systemic diseases can also cause the dry eye disease, such as systemic lupus erythematosus, diabetes mellitus, sjogren syndrome, rheumatoid arthritis, sleep disorders, a perimenopausal syndrome, a thyroid disease, and allergic conjunctivitis. Occurrence of the dry eye disease is also related to age. In addition, the dry eye disease occurs also due to many environmental factors such as environmental pollution or excessively dry environment, an eye surgery and excessive use of ocular drugs; and excessive use of video terminals, wearing of corneal contact lenses and other factors can lead to occurrence of the dry eye disease. A worker who is often engaged in outdoor work is more prone to having the dry eye disease. Although many researches have been performed on pathogenic factors of the dry eye disease, pathogenesis of the dry eye disease is not completely clear.

Loxoprofen sodium is a non-steroidal anti-inflammatory drug, which is clinically used for anti-inflammation and analgesia for rheumatoid arthritis, low back pain, scapulohumeral periarthritis, neck-shoulder-wrist syndrome and the like, analgesia and anti-inflammation after a surgery, a trauma and tooth extraction, antipyresis and analgesia for acute upper respiratory tract inflammation, etc. There is no report on treatment of the dry eye disease with the loxoprofen sodium.

### CONTENT OF THE PRESENT INVENTION

The present invention aims to overcome the defects of the prior art, and provide a drug for treating a dry eye disease with good therapeutic effect and low irritation.

Specifically, the present invention provides a use of loxoprofen sodium or a composition containing loxoprofen sodium in the preparation of a drug for preventing and/or treating a dry eye disease.

The loxoprofen sodium (chemical name: 2-[4-(2-oxocyclopentyl-1-methyl) phenyl] propionate sodium dihydrate) is a non-steroidal anti-inflammatory drug. Its mechanism of action is to inhibit prostaglandin synthesis, and its target is cyclooxygenase. At present, this compound is clinically used for anti-inflammation and analgesia for rheumatoid arthritis, low back pain, scapulohumeral periarthritis, neck-shoulder-wrist syndrome and the like, analgesia and anti-inflammation after surgery, trauma and tooth extraction, antipyresis and analgesia for acute upper respiratory tract inflammation, etc.

It is creatively discovered by the present invention that the loxoprofen sodium has the efficacy of promoting tear secretion, enhancing the tear film stability and alleviating corneal epithelial cell injury. It may be used for preventing and/or treating the dry eye disease, especially the dry eye disease related to lacrimal gland dysfunction or tear deficiency, poor tear film stability and/or corneal epithelial cell injury.

The present invention also provides a use of loxoprofen sodium or a composition containing loxoprofen sodium in the preparation of a drug for treating lacrimal gland dysfunction or promoting tear secretion.

The present invention also provides a use of loxoprofen sodium or a composition containing loxoprofen sodium in the preparation of a drug for enhancing tear film stability.

The present invention also provides a use of loxoprofen sodium or a composition containing loxoprofen sodium in the preparation of a drug for preventing and/or treating corneal epithelial cell injury.

In some embodiments, the composition containing the loxoprofen sodium further contains one or more of a thickening agent, a metal ion complexing agent, an osmotic pressure regulator, a pH regulator and a bacteriostatic agent.

In some embodiments, the composition containing the loxoprofen sodium also contains, at a minimum, a thickening agent. The composition preferably has a mass ratio of the loxoprofen sodium to the thickening agent of 1: (0.5-1.5), more preferably, the ratio is 1: (0.8-1).

In some embodiments, the thickening agent is selected from one or more of sodium hyaluronate, methylcellulose, polyvinyl alcohol, polyvinylpyrrolidone, poloxamer, hydroxypropyl methylcellulose and xanthan gum, preferably, the sodium hyaluronate.

In some embodiments, the composition containing the loxoprofen sodium also contains, at a minimum, a metal ion complexing agent. The composition preferably has a mass ratio of the loxoprofen sodium to the metal ion complexing agent of 1: (0.02-0.5), more preferably, the ratio is 1: (0.08-0.2).

In some embodiments, the metal ion complexing agent is disodium edetate.

In some embodiments, the composition containing the loxoprofen sodium also contains, at a minimum, an osmotic pressure regulator. The composition preferably has a mass ratio of the loxoprofen sodium to the osmotic pressure regulator of 1: (2-18), more preferably, the ratio is 1: (4.5-10).

In some embodiments, the osmotic pressure regulator is selected from one or more of sodium chloride, mannitol, glucose and potassium chloride, preferably, the sodium chloride.

In some embodiments, the composition containing the loxoprofen sodium also contains, at a minimum, a pH regulator. The amount of the pH regulator used in the present invention is determined as actually needed, and the pH value of a final product can be adjusted to a level suitable for human use.

In some embodiments, the pH regulator is selected from one or more of sodium hydroxide, hydrochloric acid, sodium citrate, citric acid, boric acid and borax.

In some embodiments, the composition containing the loxoprofen sodium also contains, at a minimum, a bacteriostatic agent. The composition preferably has a mass ratio of the loxoprofen sodium to the bacteriostatic agent of 1: (0.2-0.5).

In some embodiments, the bacteriostatic agent is selected from one or more of thiomersal, quaternary ammonium salts, Domiphen, chlorhexidine, chlorobutanol, nipagin and sorbic acid, preferably, ethylparaben or benzalkonium chloride.

In some embodiments, the composition contains the loxoprofen sodium, the disodium edetate and the sodium chloride.

In some embodiments, the composition contains the following components in parts by weight:

| | |
|---|---|
| loxoprofen sodium | 1; |
| disodium edetate | 0.02-0.5; and |
| sodium chloride | 2-18. |

In some embodiments, the composition contains the following components in parts by weight:

| | |
|---|---|
| loxoprofen sodium | 1; |
| disodium edetate | 0.08-0.2; and |
| sodium chloride | 4.5-10. |

In some embodiments, the composition contains the loxoprofen sodium, the sodium hyaluronate, the disodium edetate and the sodium chloride.

In some embodiments, the composition contains the following components in parts by weight:

| | |
|---|---|
| loxoprofen sodium | 1; |
| sodium hyaluronate | 0.5-1.5; |
| disodium edetate | 0.02-0.5; and |
| sodium chloride | 2-18. |

In some embodiments, the composition contains the following components in parts by weight:

| | |
|---|---|
| loxoprofen sodium | 1; |
| sodium hyaluronate | 0.8-1; |
| disodium edetate | 0.08-0.2; and |
| sodium chloride | 4.5-10. |

In some embodiments, the composition contains the loxoprofen sodium, the sodium hyaluronate, the disodium edetate, the sodium chloride, and the ethylparaben or the benzalkonium chloride.

In some embodiments, the composition contains the following components in parts by weight:

| | |
|---|---|
| loxoprofen sodium | 1; |
| sodium hyaluronate | 0.5-1.5; |
| disodium edetate | 0.02-0.5; |
| ethylparaben or the benzalkonium chloride | 0.2-0.5; and |
| sodium chloride | 2-18. |

In some embodiments, the composition contains the following components in parts by weight:

| | |
|---|---|
| loxoprofen sodium | 1; |
| sodium hyaluronate | 0.8-1; |
| disodium edetate | 0.08-0.2; |
| ethylparaben or the benzalkonium chloride | 0.2-0.5; and |
| sodium chloride | 4.5-10. |

In some embodiments, the composition containing the loxoprofen sodium is an aqueous solution. The loxoprofen sodium preferably has a concentration of 0.05-5 mg/mL in the aqueous solution, more preferably, the concentration is 0.1-2 mg/mL.

In some embodiments, the drug of the present invention is a solution such as eye drops.

In some embodiments, the drug of the present invention is a gelling agent such as an eye gelling agent.

In some embodiments, the drug of the present invention is an ointment such as an eye ointment.

### BRIEF DESCRIPTION OF THE DRAWINGS

FIG. 1 shows an effect of a composition containing loxoprofen sodium on a score of ophthalmic fluorescein sodium of an alkali burned rabbit; and
FIG. 2 shows an effect of a composition containing loxoprofen sodium on a score of ophthalmic rose bengal staining of an alkali burned rabbit.

### DETAILED DESCRIPTION OF THE PREFERRED EMBODIMENT

The following examples are used for describing the present invention, but are not used for limiting the scope of the present invention.

The following examples are used to verify the therapeutic effect of loxoprofen sodium on the dry eye disease.

In order to facilitate an experiment, an aqueous solution composition containing loxoprofen sodium is prepared in the present invention. Its composition is shown in Table 1 below (a mass of the loxoprofen sodium in Table 1 is measured by C₁₅H₁₇NaO₃).

**Table 1: Composition of Composition**

| | Loxoprofen sodium (g) | Sodium hyaluronatechlorid (g) | Sodium e (g) | Disodium edetate (g) | Ethylparab en (g) | Benzalkon ium chloride (g) | Add water for injection to a final volume (L) |
|---|---|---|---|---|---|---|---|
| Composition 1 | 50 | 50 | 425 | 5 | 15 | / | 50 |
| Composition 2 | 50 | 50 | 425 | 5 | / | 15 | 50 |
| Composition 3 | 50 | 50 | 450 | 8 | / | / | 50 |
| Composition 4 | 50 | / | 500 | 10 | / | / | 50 |

This embodiment further provides a preparation method of the above composition 1, specifically including:
(1) Sodium hyaluronate was weighed, and about 112 prescription dosage of water for injection was added to the sodium hyaluronate while stirring for swelling for more than 3 h, making the sodium hyaluronate completely dissolved;
(2) loxoprofen sodium was weighed, and dissolved with a small amount of water, and sodium chloride and disodium edetate were added with stirring to dissolve; and
(3) ethylparaben was dissolved with a small amount of water for injection, completely dissolved by boiling, and then mixed with a solution of the dissolved sodium hyaluronate, loxoprofen sodium, sodium chloride and disodium edetate, and the water for injection was added to a full volume.

The other compositions may be routinely adjusted on the basis of the preparation method for the composition 1 according to differences in components to obtain their respective preparation methods.

The above aqueous solution composition may be directly processed into eye drops. On the basis of adding the water for injection to the full volume, it is filtered through 0.22 µm microporous membrane filter for sterilization, and then sub-packed in a sterile environment with an eye drop bottle.

### Example 1: Effect on Dry Eye Disease, Caused by Alkali Burning, in New Zealand Rabbit

### 1. Modeling method

Each New Zealand rabbit was anesthetized with 1% propofol injection intravenously (1.5 ml/kg). After the animal maintained in a stable anesthesia state, topical anaesthesia was administered with the eye drop of 100 µl of 2% lidocaine hydrochloride injection. In a case that the animal was maintained in the third phase of anesthesia, a filter paper strip of about 10 mm × 5 mm was dipped in 1 mol/L NaOH solution, and then placed on a palpebral conjunctiva about 2 mm above rabbit limbal cornea; and after 90 s, conjunctiva sac was repeatedly rinsed with about 50 ml of 0.9% sodium chloride injection.

### 2. Conjunctival fluorescein staining

2 µl of 0.5% sodium fluorescein solution was dripped into the conjunctival sac of a right eye of each New Zealand rabbit using a pipette; after 5 s, excess fluorescein was rinsed with 0.9% sodium chloride injection; and conjunctival staining was observed under cobalt blue diffuse light of a slit lamp microscope. Scoring criteria for conjunctival fluorescence staining: a conjunctiva was divided into four quadrants, each quadrant was divided into four grades according to a staining degree and area: 0 score represented no staining; 1 score represented scattered punctate staining; 2 scores represented densely punctate staining 3 scores represented patchy staining, with a total of 12 scores (the left eye was not modeled, serving as a negative control eye).

### 3. Conjunctival rose bengal staining

2 µl of 1% rose bengal was dripped into the conjunctival sac of the right eye of each New Zealand rabbit using the pipette; after 5 s, excess rose bengal was rinsed with 0.9% sodium chloride injection; and conjunctival staining was observed under green light of a slit lamp microscope. The scoring criteria for the conjunctival rose bengal staining is consistent with that for conjunctival fluorescence staining.

### 4. Tear secretion volume (for both eyes)

Schirmer I test was used to measure tear secretions of New Zealand rabbits. A phenol red cotton thread was clamped with medical ophthalmic forceps, and placed on an external canthus of each New Zealand rabbit. After 60 s, a wet length of the phenol red cotton thread was measured.

### 5. Tear film break-up time (for both eyes)

2 µl of 0.5% sodium fluorescein solution was dripped into the conjunctival sac of a lower eyelid of each New Zealand rabbit using an adjustable pipette. After several times of manual blinking with constant force, the rabbit eye was opened with constant force. The cornea was observed under cobalt blue light of the slit lamp microscope. If a black area appeared in the corneal green film, it represented tear film break-up. Three measurements were taken continuously, and an average value was taken. Results are shown in Table 2.

**Table 2: Effect on Tear Film Break-Up Time of Alkali Burned Rabbit ( x̅ ±S)**

| Group | Dosage (µl/eye/day) | n | Tear film break-up time | | |
|---|---|---|---|---|---|
| | | | D1 | D5 | D9 |
| Negative control eye | 400 | 35-36 | 10.99 ± 1.20 | 11.81 ± 0.95 | 11.51 ± 0.75 |
| Model control group | 400 | 6 | 7.59±0.95## | 8.76±0.82## | 9.64±0.32## |
| Composition 1 | 400 | 6 | 7.56±0.88## | 9.90±0.17##* | 10.24±0.18##* |

| | | | | | |
|---|---|---|---|---|---|
| Note: compared with the negative control eye, # represents P < 0.05, and ## represents P < 0.01; and compared with the model control group, * represents P < 0.05, and ** represents P < 0.01 (the negative control eye is the left eye; and the model control group is given with an equal volume of 0.9%sodium chloride injection). | | | | | |

It can be seen from data in Table 2, there is no significant difference in tear film break-up time on the first day after administration with the composition 1 (D1) (P > 0.05). However, on the fifth day (D5) and the ninth day (D9) after administration, the tear film break-up time is obviously prolonged, and there is a statistical difference (P < 0.05 or 0.01).

**Table 3: Effect on Ophthalmic Score of Alkali Burned Rabbit ( x̅ ±S)**

| Group | Dosage (µl/eye/day) | | Fluorescein sodium score | | |
|---|---|---|---|---|---|
| | | n | D1 | D5 | D9 |
| Model control group | 400 | 6 | 12.0 ± 0.0 | 10.2 ± 1.3 | 5.7 ± 0.8 |
| Composition 1 | 400 | 6 | 11.3 ± 0.8 | 8.5±0.8# | 3.7±1.5# |

| | | | | | |
|---|---|---|---|---|---|
| Note: compared with the model control group, # represents P < 0.05, and ## represents P < 0.01 (the model control group is given with an equal volume of 0.9%sodium chloride injection). | | | | | |

**Table 4: Effect on Ophthalmic Score of Alkali Burned Rabbit ( x̅ ±S)**

| Group | Dosage (µl/eye/day) | n | Score of conjunctival rose bengal staining | | |
|---|---|---|---|---|---|
| | | | D1 | D5 | D9 |
| Model control group | 400 | 6 | 11.5 ± 1.2 | 9.7 ± 0.8 | 6.0 ± 0.9 |
| Composition 1 | 400 | 6 | 11.5 ± 0.8 | 8.5 ± 1.2 | 3.8±1.3## |

| | | | | | |
|---|---|---|---|---|---|
| Note: compared with the model control group, # represents P < 0.05, and ## represents P < 0.01 (the model control group is given with an equal volume of 0.9% sodium chloride injection). | | | | | |

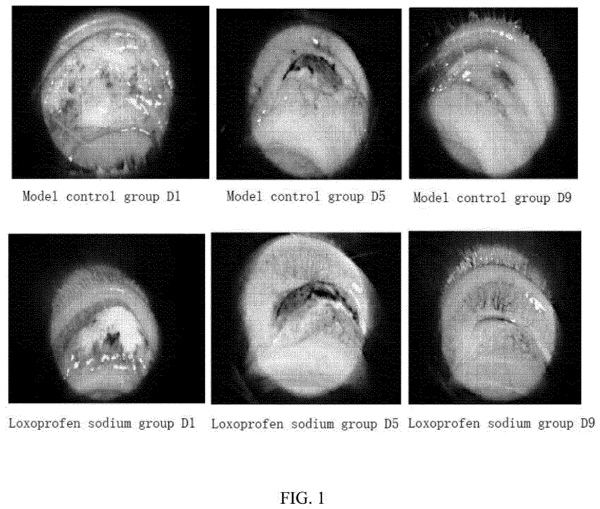

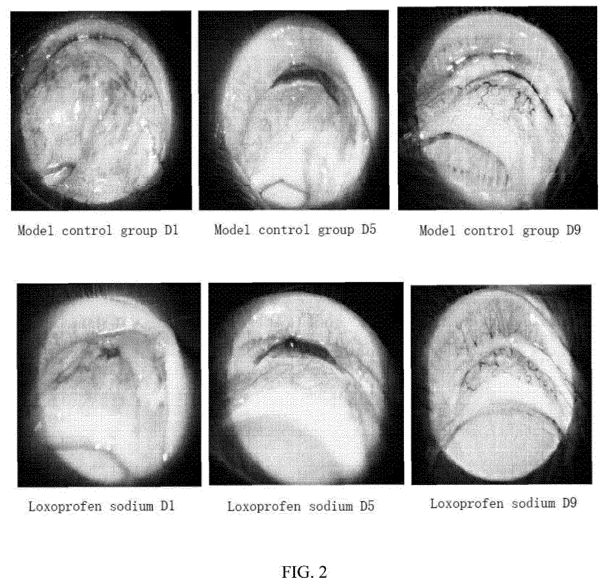

The effects of the compositions on scores of ophthalmic fluorescein sodium of an alkali burned rabbit and ophthalmic rose bengal staining of an alkali burned rabbit are shown in FIG. 1 and FIG. 2 respectively. It can be seen from data in Table 3 and Table 4 in combination with FIG. 1 and FIG. 2, compared with the model control group, the composition 1 containing the loxoprofen sodium obviously reduces the ophthalmic scores on the fifth day and the ninth day after administration. It can be seen that the composition containing the loxoprofen sodium may effectively alleviate an eye injury.

**Table 5: Effect on Wet Length of Phenol Red Cotton Thread for Alkali Burned Rabbit ( x̅ ±S)**

| Group | Dosage (µl/eye/day) | n | Wet length of phenol red cotton thread | | |
|---|---|---|---|---|---|
| | | | D1 | D5 | D9 |
| Negative control eye | 400 | 6 | 39.97 ± 5.07 | 40.05 ± 4.57 | 40.53 ± 3.01 |
| Composition 1 | 400 | 6 | 27.41±1.90## | 30.43±3.68## | 30.85±0.58## |

| | | | | | |
|---|---|---|---|---|---|
| Note: compared with the negative control eye, # represents P < 0.05, and ## represents P < 0.01. (Negative control eye is the left eye) | | | | | |

It can be seen from data in Tables 2-5, the composition containing the loxoprofen sodium obviously improves an alkali burning-induced rabbit dry eye model. Its improvement effect is mainly manifested by enhancing the tear film stability, increasing a volume of the tear secretions and alleviating the corneal epithelial cell injury (mainly manifested by reducing local edema caused by the eye injury). In addition, through a pathological research report, it is found that the composition containing the loxoprofen sodium also has the effect of increasing a number of goblet cells.

In this example, the compositions 2-4 are detected by the same method. The compositions 2-4 obviously improve the alkali burning-induced rabbit dry eye model, and there is no significant difference in effect from the composition 1.

### Example 2: Effect on Conjunctival Xerosis Syndrome in Mouse

### 1. Preparation method for antigen

Mice of a same species were euthanized by cervical vertebra dislocation, and disinfected around eyes by a conventional method. Conjunctivae were taken out on an ultra-clean bench, washed with normal saline containing 80,000/500 mL of gentamicin, and placed in the normal saline containing 80,000/500 mL of the gentamicin. The conjunctivae were cut into fragments and ground into homogenate in a homogenizer. The homogenate was crushed for 1 h on an ultrasonic crusher in a case of keeping at 4°C, and then centrifuged in a centrifuge (3000 r/min, × 15 min). A supernatant was taken out, and a content of a protein was determined by a Coomassie brilliant blue method. Then a concentration of the protein was diluted to 100 µg/mL with 0.14 MPBS. An equal volume of conjunctival antigen solution was added to a complete Freund's adjuvant (FCA), for fully mixing to obtain an water-in-oil solution.

### 2. Experimental method

The above water-in-oil solution was injected into the backs subcutaneously, axillary lymph nodes of limbs and foot pads of mice at multiple points, with a total amount of 0.5 mL. Specifically:
(1) Tear film break-up time (BUT): 1 µl of the water-in-oil solution was dripped into the lower conjunctival sac of the BALB/c mice using a micropipet. The corneas were observed under cobalt blue light of a slit lamp microscope. If black areas appeared in corneal green films, it represented tear film break-up. Three measurements were taken continuously, and an average value was taken. The BUT was normal in a case of ranging from 10 s to 15 s, and abnormal in a case of being less than 10 s. Measurement was repeated for 3 times, and an average value was taken.
(2) Schirmer test on BALB/c mice: Schinner I test was used to measure volumes of tear secretions of the BALB/c mice. A phenol red cotton thread was clamped with medical ophthalmic forceps, and placed on an external canthus of each BALB/c mouse. After 60 s, a wet length of the phenol red cotton thread was measured.
(3) Statistical analysis method: all datas were input into EXCEL for statistical analysis. Measurement data of each group was used for calculating x̅ ±S. Before comparison between the groups in various experimental groups, analysis of variance between the groups (F-test) was performed. If the variance between groups was homogeneous, Student-T test (unpaired T test) was used for comparison between groups. If the variance between groups was not homogeneous, the corrected Student-T test was used for statistical analysis.

**Table 6: Effect on Tear Film Break-up Time of Mouse Conjunctival Xerosis Syndrome Model ( x̅ ±S)**

| Group | Dosage (µl/eye/day) | n | Tear film break-up time | | |
|---|---|---|---|---|---|
| | | | D1 | D4 | D7 |
| Blank control group | 40 | 10 | 11.04 ± 0.33 | 11.96 ± 0.54 | 12.56 ± 0.73 |
| Model control group | 40 | 10 | 7.73±0.32## | 8.46±0.36## | 9.57±0.32## |
| Composition 1 | 40 | 10 | 7.88±0.21## | 10.15±0.18##** | 11.18±0.29##** |

| | | | | | |
|---|---|---|---|---|---|
| Note: compared with the blank control group, # represents P < 0.05, and ## represents P < 0.01; and compared with the model control group, # represents P < 0.05, and ## represents P < 0.01. | | | | | |

**Table 7: Effect on Wet Length of Phenol Red Cotton Thread for Mouse Conjunctival Xerosis Syndrome Model ( x̅ ±S)**

| Group | Dosage (µl/eye/day) | n | Wet length of cotton thread (mm) | | |
|---|---|---|---|---|---|
| | | | D1 | D4 | D7 |
| Blank control group | 40 | 10 | 4.30 ± 0.53 | 3.98 ± 1.03 | 3.85 ± 0.71 |
| Model control group | 40 | 10 | 2.42±0.27## | 2.74±0.51## | 2.75±0.48## |
| Composition 1 | 20 | 10 | 2.45±0.31## | 3.12±0.59##* | 3.15±0.60##* |
| Composition 1 | 40 | 10 | 2.56±0.24## | 3.23±0.88#** | 3.29±0.54##** |

| | | | | | |
|---|---|---|---|---|---|
| Note: compared with the blank control group, # represents P < 0.05, and ## represents P < 0.01; and compared with the model control group, # represents P < 0.05, and ## represents P < 0.01. | | | | | |

It can be seen from experimental data in Table 6 and Table 7, the compound 1 obviously improves the mouse conjunctival xerosis syndrome model, and its improvement effect is mainly manifested by increasing the volumes of the tear secretions in mice and prolonging the tear film break-up time.

In this example, the compositions 2-4 are detected by the same method. The compositions 2-4 obviously improve the mouse conjunctival xerosis syndrome model, and there is no significant difference in effect from the composition 1.

### Example 3 Irritation Experiment

### 1. Source of eye drops

Pranoprofen eye drops: commercially available, manufactured by Senju Pharmaceutical Co., Ltd. Fukusaki Plant.

Diclofenac sodium eye drops: commercially available, manufactured by Zhengzhou Zhuofeng Pharmaceutical Co., Ltd.

Loxoprofen sodium eye drops: it is obtained by filtering the composition 1 with a 0.22 µm microporous membrane.

2. Experimental operation: 20 patients with the dry eye disease were selected from each group, and the patients were administered three kinds of eye drops in a randomized and double-blinded manner, to compare irritative symptoms of the pranoprofen eye drops, the diclofenac sodium eye drops and the loxoprofen sodium eye drops with concentrations of 0.1%. Eye irritative symptoms include burning sensation, tingling sensation, tears, etc. Statistic results are shown in Table 8.

**Table 8: Irritation Comparison Experiment Record**

| Group | Dosage | Number of people with irritative symptoms | | | | Proportion of people with symptoms |
|---|---|---|---|---|---|---|
| | | Burning | Tingling sensation | Tears | No symptom | |
| Pranoprofen | 2 drops/time | 1 | 2 | 1 | 16 | 20% |
| Diclofenac sodium | 2 drops/time | 2 | 3 | 3 | 12 | 40% |
| Loxoprofen sodium | 2 drops/time | 0 | 0 | 1 | 19 | 5% |

According to the results of preliminary statistics, at least one eye irritative symptom appears in 40% of people in the diclofenac sodium eye drop group. At least one eye irritative symptom appears in 20% of people in the pranoprofen eye drop group. However, in the loxoprofen sodium eye drop group, only one patient has the eye irritative symptom which is a less irritative tear symptom; other patients generally reflect that after use, their eyes strongly feel comfortable, so the loxoprofen sodium eye drops may be used for a long time, and are also more suitable for children with the dry eye disease to use.

In this example, the compositions 2-4 are detected by the same method. It is found that irritation of the eye drops provided by the compositions 2-4 is less.

Although the present invention has been described in detail above with a general description, specific embodiments and tests, some modifications or improvements can be made on the basis of the present invention, which is apparent to those skilled in the art. Therefore, all these modifications or improvements made without departing from the spirit of the present invention fall within the scope of the present invention.

## Claims

1. A use of loxoprofen sodium or a composition containing loxoprofen sodium in the preparation of a drug for preventing and/or treating a dry eye disease.

2. The use according to claim 1, wherein the dry eye disease is caused by lacrimal gland dysfunction or reduced tear secretion, poor tear film stability and/or corneal epithelial cell injury.

3. The use according to claim 1, wherein the drug is to prevent and/or treat the dry eye disease by promoting tear secretion, enhancing the tear film stability and/or alleviating the corneal epithelial cell injury.

4. A use of loxoprofen sodium or a composition containing loxoprofen sodium in the preparation of a drug for treating lacrimal gland dysfunction or promoting tear secretion.

5. A use of loxoprofen sodium or a composition containing loxoprofen sodium in the preparation of a drug for enhancing tear film stability.

6. A use of loxoprofen sodium or a composition containing loxoprofen sodium in the preparation of a drug for preventing and/or alleviating corneal epithelial cell injury.

7. The use according to any one of claims 1-6, wherein except the loxoprofen sodium, the composition further contains one or more of a thickening agent, a metal ion complexing agent, an osmotic pressure regulator, a pH regulator and a bacteriostatic agent.

8. The use according to claim 7, wherein the thickening agent is selected from one or more of sodium hyaluronate, methylcellulose, polyvinyl alcohol, polyvinylpyrrolidone, poloxamer, hydroxypropyl methylcellulose and xanthan gum; and/or,
the metal ion complexing agent is selected from disodium edetate; and/or,
the osmotic pressure regulator is selected from one or more of sodium chloride, mannitol, glucose and potassium chloride; and/or,
the pH regulator is selected from one or more of sodium hydroxide, hydrochloric acid, sodium citrate, citric acid, boric acid and borax; and/or,
the bacteriostatic agent is selected from one or more of thiomersal, quaternary ammonium salts, Domiphen, chlorhexidine, chlorobutanol, nipagin and sorbic acid.

9. The use according to claim 8, wherein the composition contains the loxoprofen sodium, the disodium edetate and the sodium chloride.

10. The use according to claim 9, wherein the composition contains the following components in parts by weight:
| | |
|---|---|
| loxoprofen sodium | 1; |
| disodium edetate | 0.02-0.5; and |
| sodium chloride | 2-18. |

11. The use according to claim 8, wherein the composition contains the loxoprofen sodium, the sodium hyaluronate, the disodium edetate and the sodium chloride.

12. The use according to claim 11, wherein the composition contains the following components in parts by weight:
| | |
|---|---|
| loxoprofen sodium | 1; |
| sodium hyaluronate | 0.5-1.5; |
| disodium edetate | 0.02-0.5; and |
| sodium chloride | 2-18. |

13. The use according to claim 8, wherein the composition contains the loxoprofen sodium, the sodium hyaluronate, the disodium edetate, the sodium chloride, and ethylparaben or benzalkonium chloride.

14. The use according to claim 13, wherein the composition contains the following components in parts by weight:
| | |
|---|---|
| loxoprofen sodium | 1; |
| sodium hyaluronate | 0.5-1.5; |
| disodium edetate | 0.02-0.5; |
| ethylparaben or the benzalkonium chloride | 0.2-0.5; and |
| sodium chloride | 2-18. |

15. The use according to any one of claims 1-14, wherein the composition containing the loxoprofen sodium is in the form of an aqueous solution.

16. The use according to claim 15, wherein the loxoprofen sodium has a concentration of 0.05-5 mg/mL in the aqueous solution.

17. The use according to claim 16, wherein the loxoprofen sodium has a concentration of 0.1-2 mg/mL in the aqueous solution.

18. The use according to any one of claims 1-17, wherein the drug is a solution, a gelling agent or an ointment.
